(19) **Europäisches Patentamt**
**European Patent Office**
**Office européen des brevets**

(11) **EP 2 086 356 B1**

(12) **EUROPÄISCHE PATENTSCHRIFT**

(45) Veröffentlichungstag und Bekanntmachung des Hinweises auf die Patenterteilung:
**02.06.2010 Patentblatt 2010/22**

(21) Anmeldenummer: **07817565.0**

(22) Anmeldetag: **24.09.2007**

(51) Int Cl.:
*A23L 1/305* (2006.01)  *A23J 1/00* (2006.01)
*A23J 1/12* (2006.01)  *A23J 1/14* (2006.01)
*A23K 1/14* (2006.01)  *C07K 1/30* (2006.01)
*C07K 14/415* (2006.01)  *A61K 8/64* (2006.01)

(86) Internationale Anmeldenummer:
**PCT/DE2007/001723**

(87) Internationale Veröffentlichungsnummer:
**WO 2008/052501 (08.05.2008 Gazette 2008/19)**

(54) **VERFAHREN ZUM ERHALT VON KNOLLENFRUCHTPROTEINFRAKTIONEN MITTLEREN MOLEKULARGEWICHTS, KNOLLENFRUCHTPROTEINFRAKTION UND VERWENDUNG DERSELBEN**

METHOD OF OBTAINING A TUBEROUS CROP PROTEIN FRACTIONS WITH A MEDIUM MOLECULAR WEIGHT, TUBEROUS CROP PROTEIN FRACTION, AND ITS USE

PROCÉDÉ D'OBTENTION DE FRACTIONS DE PROTÉINES DE PLANTES A TUBERCULES DE POIDS MOLÉCULAIRE MOYEN, FRACTION DE PROTÉINE DE PLANTES A TUBERCULES ET SON UTILISATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.10.2006 DE 102006050620**

(43) Veröffentlichungstag der Anmeldung:
**12.08.2009 Patentblatt 2009/33**

(73) Patentinhaber: **Emsland-Stärke GmbH**
**49824 Emlichheim (DE)**

(72) Erfinder:
• **LOTZ, Martin**
**49824 Emlichheim (DE)**
• **EGGENGOOR, Gerold**
**49849 Wilsum (DE)**

(74) Vertreter: **Neidl-Stippler, Cornelia**
**Neidl-Stippler**
**Patentanwaltskanzlei**
**Rauchstrasse 2**
**81679 München (DE)**

(56) Entgegenhaltungen:
**WO-A-2006/047308    DE-A1- 2 922 247**
**FR-A- 2 876 389**

• **CHANGO ET AL.: "Fractionation by thermal coagulation of lupin proteins: physicochemical characteristics" FOOD RESEARCH INTERNATIONAL, Bd. 28, Nr. 1, 1995, Seiten 91-99, XP002472356 ISSN: 0963-9969**
• **RALET ET AL.: "Fractionation of potato proteins: solubility, thermal coagultion, and emulsifying properties" LEBENSMITTEL-WISSENSCHAFT AND TECHNOLOGIE, Bd. 33, Nr. 5, 2000, Seiten 380-387, XP002472357 ISSN: 0023-6438**
• **LINDNER ET AL.: "Fractionation of potato juice protein into acid-soluble and acid coagulable fractions" FOOD CHEMISTRY, Bd. 6, Nr. 4, 1981, Seiten 323-335, XP002472358 ISSN: 0308-8146**

EP 2 086 356 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Verfahren nach dem Oberbegriff des Patentanspruches 1 eine Knollenfruchtproteinfraktion nach dem Oberbegriff des Patentanspruches 5 und deren Verwendung nach Anspruch 7. Sie bezieht sich also auf die Gewinnung von pflanzlichen Proteinen, die pflanzlichen Proteine selbst sowie die Verwendung dieser Proteine.

Proteine sind lebenswichtige chemische Verbindungen für die gesamte lebende Welt, meist mit biochemischer Funktion als Enzyme, aber auch als Speicherstoffe (Speicherproteine), sozusagen als Rohstoffreservoir für lebenswichtige Vorgänge, vor allem bei Wachstum bzw. Vermehrung. Proteine sind durch ihre Molekülgröße, ihre Zusammensetzung sowie ihre Sekundär- und Tertiärstruktur gekennzeichnet. Von Proteinen oder Eiweiß spricht man dann, wenn der chemische Aufbau ausschließlich aus Aminosäuren besteht und die Kettenlänge ca. 30 Aminosäuren und mehr beträgt. Bei kürzeren Ketten spricht man im Allgemeinen von Peptiden, ohne dass diese Abgrenzung genau definiert wäre. Sie ist eher willkürlich und für bestimmte Situationen nützlich. Proteine werden von einzelligen Lebewesen, z.B. Bakterien und Hefen, Pflanzen, aber auch von Tieren erzeugt. Für die menschliche und tierische Ernährung und Gesundheit sind sie unverzichtbar. Neben ihren chemischen, nutritiven und biochemischen Eigenschaften haben Proteine auch sogenannte funktionale Eigenschaften, von denen die Wasseraufnahmekapazität, Verdaulichkeit, Löslichkeit in Wasser, die Bildung und Stabilisierung von Schaum sowie die Emulsionsfähigkeit für ihre technische Anwendbarkeit herausragenden Merkmale sind, die auch durch die tertiäre und sekundäre Struktur bestimmt sind. Dadurch finden Eiweiße auch in der so Technik breite Anwendung, unter anderem als Kleber, Emulgatoren, Andickungsmittel. Die tertiäre Struktur wird durch Hitzeeinwirkung oder aber durch Einwirkung von Säuren oder Laugen schwerwiegend und häufig irreversibel gestört. Die Sekundärstruktur wird durch Spaltung der Proteine, wie sie durch Enzyme oder aber stark alkalische oder saure Medien erfolgt, zerstört. Es ist somit wesentlich, funktionale Eigenschaften der Proteine während der Isolation derselben aufrecht zu erhalten.

Stand der Technik ist, tierische sowohl als auch pflanzliche Proteine zu isolieren. Sie werden bereits vielfältig angewendet, z.B. in Lebensmitteln (Tofu), Futtermitteln, der Pharmazie und in technischen Bereichen (Eiweißkleber od. dgl.) Die Bedeutung der Proteine für Ernährung, Lebensmitteltechnologie, z.B. als Schaummittel, Emulgator, Strukturgeber bzw. Texturierer (z.B. Gelatine für Gummibärchen und Tortenguss), Tierfutter, Kosmetika und Arzneimittel ist einzigartig und kann durch keine andere Stoffgruppe ersetzt werden. Am einfachsten sind funktionelle Proteine, die gute Wasserlöslichkeit und Emulsionsfähigkeit aufweisen, aus Milch oder Eiern durch wenig belastende Prozesse (keine starken pH-Wert-Änderungen, keine hohe Erhitzung) erhältlich. Im technischen Bereich findet sehr verbreitet Caseinat als Etikettierleim für Flaschenetiketten Verwendung, als Kosmetikum ist Collagen weit verbreitet.

Problematisch ist, dass insbesondere tierische Proteine häufig allergische Reaktionen auslösen.

Die sehr häufig industriell eingesetzten Kuhmilchproteine sind bei vielen Verbrauchern, die eine Lactoseintoleranz oder eine Allergie gegen Kuhmilchproteine aufweisen, gefürchtet. Tierische Proteine haben noch dazu immer den Nachteil, dass sie Träger von Krankheiten (wie BSE, HIV oder aber Vogelgrippe) tragen oder pathogen sein können. Die Verwendung tierischer Proteine hat auch den Nachteil, dass diese in vielen Bevölkerungsgruppen aus ethischen Gründen nicht akzeptiert werden. Hautcremes auf Collagenbasis etwa sind aus diesem Grund im asiatischen und moslemischen Kulturkreis verpönt, führen aber auch in unseren Kulturen zu Allergien. Auch sind tierische Proteine in aller Regel teurer als pflanzliche Proteine, da die Nachhaltigkeit pflanzlicher Proteine wesentlich höher als bei tierischen Proteinen ist - sie sind preiswerter zu produzieren und außerdem für eine vegetarische oder andere Diät, wie bspw. eine purinarme Diät oder Ernährung besonders geeignet. Dies macht es sinnvoll, pflanzliche Proteine näher zu untersuchen.

Pflanzenproteine, insbesondere das hochwertige Kartoffelprotein vermeidet viele der oben genannten Nachteile der tierischen Proteine. Viele sind wenig bis nicht allergen - d.h. z.B. nicht auf der Allergenliste der EU geführt, als pflanzliches Protein in allen Kulturen akzeptiert und aufgrund der Kultur bestimmter Knollenfrüchte, wie von Kartoffeln, ist die Sicherstellung gentechnikfreier Produkte (non-GMO-Zertifikate) und von Bio-Produkten möglich.

Von allen industriell verwendeten tierischen Proteinen wie etwa Milch- und Molkeproteine, Gelatine, Hühnereiweiß, Collagen usw. sind im technischen Einsatz im allgemeinen - d.h. in der Lebensmitteltechnologie, der Technik u. dgl. fast ausschließlich Milchproteine, vor allem Casein und seine Salze, Hühnereiweiß als Volleiweiß oder Eiklar sowie Eigelb und aus Schlachtabfällen isolierte Proteine, wie Gelatine, Knochenleim und Collagen. Isolierte pflanzliche Proteine sind bis heute nur aus wenigen Pflanzen in der täglichen Anwendung verbreitet.

Gebräuchliche isolierte Pflanzenproteine stammen von Leguminosen, wie Soja, eingeschränkt auch von Erbsen und Weizen. In größerem Umfang sind es als Isolate nur Soja- und Weizenprotein, der sogenannte Gluten. Gluten ist ein problematisches Eiweiß, da viele Menschen unter einer Glutenallergie leiden (Zöliakie) und ein erheblicher Bedarf an glutenfreiem pflanzlichem Eiweiß besteht. Sojaprotein, das in großem Umfang als Austauschstoff für tierisches Eiweiß sowie als Tierfuttermittel weit verbreitet ist, ist nicht unumstritten, da es hormonaktive Substanzen enthält.

Weitere isolierte Pflanzenproteine untergeordneter Bedeutung stammen aus Raps, Lupinen und anderen Leguminosen oder Kartoffeln. Die Qualität vieler kommerziell erhältlicher isolierter Pflanzenproteine, insbesondere der Kartoffelproteine ist nicht zufriedenstellend, obwohl dies wünschenswert wäre. Die Gründe hierfür sind verschieden.

Nachfolgend wird die Erfindung anhand von Kartoffeln näher erläutert - sie ist jedoch keinesfalls auf diese Spezies eingeschränkt, sondern kann, wie dem Fachmann offensichtlich, leicht für andere Knollenfrüchte und Pflanzen angepaßt werden.

Für die Herstellung von Pflanzenprotein aus Fruchtwasser, das durch Pressen der entsprechenden Pflanzenteile, insbesondere Früchte, oder durch Extraktion aus Pflanzenteilen gewonnen wird, werden technisch prinzipiell zwei Verfahren eingesetzt:

1. Wärmekoagulation der Proteine im Fruchtwasser unter Inaktivierung der Enzyme oder

2. Ausfällung der Proteine aus dem Fruchtwasser bei saurem pH.

Unter Knollenfruchtteilen werden im Zusammenhang mit der Erfindung die Speicherorgane von Knollenfrüchten (z.B. Kartoffeln, Tapioka, Topinambur, Yams, Süßkartoffel, Taro, Bitterer Maniok, Yams, Papyrus, Ullucus tuberosus C. - Knollige Kapuzinerkresse, Oxalis tuberosa M. etc.) verstanden.

Unter Fruchtwasser werden im Zusammenhang sowohl aus den Pflanzenteilen ausgepresste Säfte als auch durch Extraktion mit wässrigen Medien aus den Pflanzenteilen gewonnene Lösungen von Pflanzenproteinen verstanden, die dann durchzuführen ist, wenn das Pflanzenteil keinen ausreichenden Flüssigkeitsgehalt besitzt oder aber um verbliebenes Protein zu mobilisieren.

Bisher wurden derartige Pflanzenproteine klassisch durch Hitze/Säurebehandlung des Fruchtwassers_und Abtrennen des ausgefällten Proteins direkt aus dem Fruchtwasser gewonnen.

Bei der klassischen Hitzefällung der Proteine aus dem Fruchtwasser entsteht ein schwerlösliches Produkt unzureichender Funktionalität, das schlecht verdaulich ist, starken Nebengeschmack hat und noch dazu mit Schadstoffen behaftet ist. Bei höherer Temperatur wird das Protein übermäßig geschädigt und seine wertvollen Eigenschaften, die es für die Lebensmittelanwendung so nützlich machen werden zunehmend zerstört: neutraler Geschmack, helle Farbe, Löslichkeit, alle anderen Funktionalitäten ebenfalls, die Struktur wird verhornt und die Verdaulichkeit nimmt ab.

Typische Negativstoffe, die als Begleitstoffe in gefällten Pflanzenproteinen auftreten, sind bspw: Trypsininhibitoren, Proteine, welche das Eiweißverdauungsenzym Trypsin und damit die Verdauung hemmen. Trypsininhibitor kann nur durch gezielte Deaktivierung unschädlich gemacht werden, z.B. durch eine Hitzebehandlung von 70 °C oder höher.

Ferner können unter anderem auftreten: Tannine/Gerbsäuren, hindern die Verdauung, die Eisenaufnahme und inaktivieren Verdauungsenzyme; toxische Glycoalkaloide, wie Solanin in Nachtschattengewächsen; Proteaseinhibitoren und Polyphenole.

Viele Pflanzenproteine, die durch Hitzefällung einer Proteinlösung im alkalischen Milieu gewonnen werden, (wozu übrigens auch die ggf. notwendige Pasteurisierung zählt), haben noch dazu einen hohen Gehalt an Lysinoalanin, einem antinutritiven Kondensationsprodukt, dessen Gehalt grundsätzlich so gering wie nur möglich sein soll.

Bei den z.Zt. erhältlichen pflanzlichen Proteinen ist auch der starke Eigengeschmack ein Problem; wie z.B. bei Sojaprotein, wodurch viele Anwendungen nicht möglich sind oder die Einsatzmenge limitiert ist, was ebenfalls oft nachteilig ist.

Für Nahrungsmittelzwecke ist zu beachten, dass die meisten Pflanzenproteine nicht vollwertig sind, d.h. dass sie nicht alle Aminosäuren, insbesondere die 8 sog. essentiellen Aminosäuren, die der menschliche Körper nicht selbst herstellen kann und daher extern zugeführt bekommen muss, enthalten. Die Wertigkeit von Pflanzenproteinen ist geringer als die von tierischen Proteinen - mit am besten in der Wertigkeit schneiden bei pflanzlichen Proteinen Kartoffelproteine ab.

Es ist bspw. bereits Kartoffelprotein erhältlich, das durch scharfe Koagulation bei hohen Temperaturen und Trocknung hergestellt wird und eine Zulassung nach der Novel Food-VO besitzt,. Hydrolysate aus diesem Kartoffelprotein sind ebenfalls nach der Novel Food-VO zugelassen. Nachteile dieses bekannten Proteins sind seine relativ dunkle Farbe, sein Geschmack nach Kartoffeln und der scharfe "brenzlige" bis bittere Geschmack. Dies ist auf eine starke Denaturierung der Proteine, Änderung der Tertiärstruktur und Verlust von technologische Funktionalität, u. a. der Bindungsfähigkeit des genannten Kartoffelproteins für Wasser und/oder Öle zurückzuführen. Man hat daher versucht, durch Hydrolyse dieses Kartoffelproteins die Löslichkeit und damit technologische Funktionalität zurück zu erhalten, hat dann aber wieder Probleme, die typisch für Proteinhydrolysate aller Provenienz sind, nämlich Allergenität sowie bitterer Geschmack durch Peptide und natürlich den durch die Mehrfachbehandlung hohen Aufwand, der zu sehr hohen Herstellkosten und damit verbundenen hohen Marktpreise.

Ein weiterer Nachteil der bislang bekannten Verfahren zur Isolation von Kartoffelproteinen ist, dass die für den menschlichen und tierischen Genuss toxischen Glycoalkaloide, von denen das Solanin am bekanntesten ist, in zusätzlichen Verfahrensschritten extra abgetrennt werden müssen. Dies geschieht durch gezielte Auswaschung mit viel Waschwasser in saurem Milieu bei nur wenig Trockensubstanz, da die Glycoalkaloide nur eine geringe Löslichkeit besitzen, oder aber man benutzt teure Lösungsmittel, die wiedergewonnen und aufgearbeitet werden müssen. Beide Verfahren sind sehr aufwendig. Die braune Farbe der bekannter Kartoffelproteine, hervorgerufen durch zu Polychinonen und Melaminen oxidierte Polyphenole, die nebenbei für den bitteren Geschmack verantwortlich sind, ist als ein weiterer und entscheidender Nachteil zu nennen, der bislang verhindert hat, dass das prinzipiell hochwertige Kartoffelprotein im Nahrungsbereich verwendet wird.

Diese durch thermische Fällung hergestellten bekannten Kartoffelproteine weisen dazu geringe Funktionalität und aufgrund ihrer hohen Denaturierung schlechte physiologische Verwertbarkeit auf, sind also schlecht zu verdauen und eignen sich schlecht bis gar nicht als Milchproteinersatzstoffe. Ähnliches gilt für die Proteine anderer Pflanzen, wie Weizen, Raps, Soja etc., bei denen schädliche Nebenstoffe vorliegen.

Es ist Aufgabe der Erfindung, technisch wenig aufwendig Knollenfruchtproteine besserer Funktionalität bereitzustellen, die Nachteile bekannter Knollenfruchtproteine vermeiden.

Die Aufgabe wird erfindungsgemäß durch ein Verfahren mit den Merkmalen des Patentanspruches 1 gelöst. Ferner bezieht sich die Erfindung auf eine Proteinfraktion mit den Merkmalen des Patentanspruches 5 sowie die Verwendung der Proteinfraktion nach Anspruch 7. Vorteilhafte Weiterbildungen ergeben sich aus den Unteransprüchen.

Erfindungsgemäß wird also durch gezielte Fraktionierung des Proteins mit proteinschonenden, überraschend einfachen Verfahren eine Proteinfraktion lebensmitteltauglicher Qualität mit ausreichender Funktionalität erhalten. Wesentlich ist, daß die Fraktionierung als Trenntechnik für verschiedene Proteine eingesetzt wird. Die Fraktionierung durch gezielte Einstellung von pH-Wert und Temperatur ist ein effizientes und preiswertes Verfahren, was eine einfache überraschend gezielte Proteinfraktionierung im technischen Großmaßstab ermöglicht. Die erfindungsgemäße Fraktion kann auch durch stufenweise Membranfiltration oder stufenweise Fällung mit Lösungsmitteln erhalten werden, aber erheblich aufwendiger. Es ist häufig günstig, die mechanische Abtrennung mit Dekantem durchzuführen, die schnell große Mengen Material in Feststoffe und Überlauf kontinuierlich trennen können.

Bei vielen Knollenfruchtteilen - bspw. Kartoffeln - ist eine "anoxische Prozessführung", sinnvoll, zumindest bis die Polyphenole (PP) von der gewünschten Proteinfraktion (praktisch heißt das, aus dem Knollenfruchtsaft) abgetrennt und/oder die Proteine, die die enzymatische Oxidation katalysieren, koaguliert sind und somit keine enzymatisch katalysierten und/oder nichtenzymatisch katalysierten oxidierenden Prozesse mehr ablaufen können. Die Durchführung der anoxischen Prozessführung kann durch Ausschluss von Luftsauerstoff z.B. durch Eintrag von Stickstoff, Rauchgasen unter anderem Gasen, erfolgen, wodurch die Luft aus Material, Rohren und Apparaten zu verdrängt wird, durch Einsatz luftdichter Apparaturen, die nur den natürlichen Sauerstoffgehalt der Knollenfruchtteile im Prozess zulassen, Abfangen des im Prozess enthaltenen Sauerstoffs durch Hilfschemikalien wie Antioxidantien (Ascorbinsäure, Zitronensäure, $SO_2$, Natriumbisulfit unter anderem) und/oder Entschäumer. Natürlich können alle 3 genannten Prinzipien und/oder angewandt werden in beliebiger Kombination.

Die erfindungsgemäße Knollenfruchtproteinfraktion eignet sich besonders als Lebensmittel, Nahrungsmittelzusatz, Arzneimittelzusatz, Tierfutter, in der Kosmetik, als technisches Eiweiß, als Kleber, da sie einerseits in ausreichender Menge vorliegt und andererseits eine ausreichende Funktionalität aufweist.

Die gezielte Fraktionierung von Knollenfruchtprotein, wie es bspw. im nativen Kartoffelsaft/Kartoffelfruchtwasser vorliegt, erfolgt mit der doppelten Zielsetzung, zunächst unerwünschte und störende Fraktionen aus dem Fruchtsaft (vorab) zu entfernen und anschließend die gewünschte, und nur ganz genau diese, Fraktion aus dem Fruchtsaft zu isolieren. Erfindungsgemäß geht man so vor, dass von Verfahrensschritt zu Verfahrensschritt die Fällungsbedingungen verschärft werden, um so jeweils die Fraktion mit den Proteinen, die das nächst niedrigere Molekulargewicht besitzen, isolieren zu können.

Das erfindungsgemäße Verfahren weist auf:

- Zerreiben der ganzen oder geschälten oder anders geteilten Frucht, um den in den Zellen eingeschlossenen Fruchtsaft freizusetzen, ggf. unter Zusatz von (wässrigem) Lösemittel;

- vollständiges oder teilweises Abtrennen des so freigesetzten Fruchtsaftes bzw. Proteinlösung, der das gesamte Protein enthält,

- mechanisches Abtrennen einer ersten unlöslichen Fraktion des Proteins nach einer ersten Fällung. Dies kann direkt aus dem Fruchtwasser erfolgen, nach Einstellung eines bestimmten pH-Wertes, einer bestimmten, nur gering über der Raumtemperatur liegenden Temperatur oder eine Kombination aus beidem. Bspw. adsorbieren bei Kartoffeln die Glycoalkaloide am so abgetrennten Protein, reichern sich in dieser abgetrennten Fraktion an und in der flüssigen Phase ab, wobei Protein niedrigeren Molekulargewichts im Überstand verbleibt. Dadurch wird eine aufwendige Abtrennung von unerwünschten Schadstoffen durch Adsorption in dieser Fraktion vermieden.

- mechanisches Abtrennen der Zielfraktion des Proteins aus dem Überstand der ersten Fällung. Die Fällung der Zielfraktion kann durch Einstellung eines geeigneten pH-Wertes und/oder einer gegenüber der ersten Fällung erhöhten Temperatur erreicht werden.

- ggf. Waschen und Trocknen der Zielproteinfraktion.

Stufe 1

Der erste Schritt dient der Abtrennung großer Proteine.. In dieser Fraktion sind auch die sogenannten Polyphenole enthalten, deren enzymatische und anschließende nichtenzymatische Oxidation zu Braunfärbung und bitterem Geschmack führt. Bei Kartoffeln ist diese Fraktion ein Schlamm, der Glycoalkaloide, dunkle unerwünschte Farbe, Kartoffelgeschmack, Polyphenole, Proteinkomplexe aufweist und allenfalls als Tierfutter geeignet ist. Der Rohproteingehalt (N*6,25) ist gering (ca. 45 %).

Dies kann man erreichen durch:

- einfaches Zentrifugieren des reinen Fruchtsaftes. Hierbei wird am wenigsten Protein abgetrennt, dafür aber "sortenrein"
oder

- Einstellen eines sauren pH-Wertes zwischen pH 2 bis 7, bevorzugt zwischen 4 - 6 und Säurefällung der großen Proteine, wobei das Ausgefällte dann mechanisch, bspw. durch Zentrifugation, abgetrennt wird. Wichtig ist, dass nicht zuviel Ausfällung (was die sowieso schon geringe Ausbeute noch mehr erniedrigt), und nicht zu wenig (was die Proteinreinheit der "Wunschfraktion" und andere Qualitätsparameter verschlechtert) auftritt
oder

  - ♦ Durch Einstellung einer gegenüber der Raumtemperatur gering erhöhten Temperatur von 25 bis 50 °C

- Durch eine angemessene Kombination aus erhöhter Temperatur (25 - 50 °C) und eines sauren pH-Wertes von 2 bis 7.

Ein besonderer Vorteil dieses Verfahrensschrittes ist seine außerordentliche Einfachheit, dies auch bezüglich Maschinen, Materialien und Energieverbrauch.

Die sicherste und preiswerteste Variante, diese unerwünschte Proteinfraktion nicht in der gewünschten Fraktion zu haben ist, sie vorab aus dem Fruchtsaft abzutrennen und dies unter definierten Prozessbedingungen zu tun, nämlich pH-Wert und Temperatur einstellen und kontrollieren. Dieser erste Schritt dient der Polyphenol- und Glycoalkaloidabtrennung sowie minderwertigen Proteinfraktionen, wobei man einen unvermeidlichen Proteinverlust billigend in Kauf nimmt.

Stufe 2

Hier wird die Zielproteinfraktion durch Einstellen eines zur Fällung geeigneten pH-Wertes in Kombination mit einer Wärmefällung aus dem Überstand des Schrittes 1 isoliert. Dazu wird ein pH-Wert im Bereich des isoelektrischen Punktes des Proteins gewählt, in Kombination mit Erhöhung der Temperatur über Raumtemperatur hinaus.

Dieser entspricht wiederum den Bedingungen der Fällung aus dem Sauren bei erhöhter Temperatur - es ist bei einem pH zwischen 2 - 6 und 50 bis 85 °C zugänglich.

Zu den Produktmerkmalen ist ergänzend zu sagen:

Überraschenderweise kann durch das erfindungsgemäße Verfahren, das keine teuren oder komplizierten Verfahren oder Prozessstufen benötigt und sehr schnell ist, mit einer Kombination einfacher Verfahrensschritte das geforderte Ziel erreicht werden, insbesondere sind die notwendigen Apparaturen auch noch preiswert in der - Anschaffung und im Betrieb.

Sehr vorteilhaft ist, dass keine chemischen Zusätze im Verfahren angewandt werden müssen, wie z.B. Enzyme, Desinfektionsmittel; Aufheller.

Überraschend ist auch hinsichtlich der Produktmerkmale bzw. -eigenschaften, dass durch die Fraktionierung nicht nur Proteine mit einem besonderen Molekulargewichtsbereich isoliert werden, sondern auch eine Fraktion, die alle geforderten Eigenschaften besitzt: hoch nutritiv, neutrale Farbe, keinen ausgeprägten Pflanzen-, bspw. Kartoffelgeschmack, geringe Lysinoalaningehalte, wenig Glycoalkaloide sowie technologische Funktionalitäten, wie Wasserbindung und Ölbindung (Emulgator). Dies ist besonders überraschend, da die gefällte Proteinfraktion fast wasserunlöslich ist.

Nachstehend wird die Erfindung nunmehr anhand von Ausführungsbeispielen, auf die sie keineswegs eingeschränkt ist, näher erläutert.

Die Prozessbedingungen für die erste Stufe, den ersten Fraktionierungsschritt, sind: Raumtemperatur bis 50 °C, pH 2 - 7, Separator als Zentrifuge. Diese so abgetrennte Proteinfraktion mit einem MG von ca. 100 - 600 kD eignet sich bspw. als gewöhnliches Tierfutter, so wie z.Zt. das meiste, unaufgetrennte Kartoffelprotein verwendet wird. Typischerweise werden unter diesen Bedingungen gemeinsam mit dem hochmolekularen Kartoffelprotein mit nicht proteinartigen Molekülen verknüpfte Proteine wie Glycoproteine, Phosphoproteine, Lipoproteine, Metall-komplexierte Proteine usw. aber auch der größte Anteil Glycoalkaloide und Polyphenole abgetrennt.

In der zweiten Stufe erfolgt das Abtrennen der Zielfraktion durch: Fällung bei pH 2 - 6, bevorzugt pH 3,5 bis 5,5 und 50 - 85 °C, bevorzugt 75 bis 85 °C, Abtrennung der ausgefällten Kartoffelproteinfraktion aus dem Kartoffelsaft mit Dekanterzentrifuge. Eine Eigentümlichkeit des Kartoffelproteins ist es, dass umso mehr Protein ausgefällt wird, je saurer der pH-Wert eingestellt wird. Bei allen anderen Proteinen, pflanzlich sowohl als auch tierischen Ursprungs, ist die Fällung am isoelektrischen Punkt maximal und auch von der "Flockenbeschaffenheit" her optimal. Bei Kartoffelprotein im Kartoffelsaft ist dies pH 5,4, was fast der native pH von Kartoffelsaft ist (pH 5,6).

Kartoffelprotein hat noch eine zweite Eigentümlichkeit: Hitzekoagulation, oberhalb von 40 °C, ist bei Kartoffelprotein irreversibel. Die Parameter gewährleisten auch die mikrobiologische Reinheit des Produkts, es ist also keine zusätzliche Pasteurisierung notwendig. Man benutzt keine hohe bis sehr hohe Temperatur und saures Milieu, daher entsteht wenig Lysinoalanin.

Das so erhaltene Proteinprodukt besaß einen Proteingehalt von ca. 75 % in Trockensubstanz

Eine Reinigung dieser Roh-Proteinfraktion mit einem MG von 14 bis 97 zu Isolatqualität, d.i. > 80, bevorzugt > 85 % Protein in Trockensubstanz, erfolgt durch Waschen mit Leitungswasser, kalt (Raumtemperatur) oder heiß (bevorzugt 60 °C bis 85 °C), pH neutral oder angesäuert auf pH 4 bis 7, d.h. Bedingungen nahe der Fällungsbedingungen aber etwas milder. Eine typische Ausführung erfolgt in mehreren Stufen, bspw. in Reihe geschalteter Dekanter, wobei vor jedem Dekanter die Hälfte des Waschwassers zugeführt wird oder im Gegenstrom, d.h. das gesamte Waschwasser wird vor dem 2. Dekanter zugeführt und im Oberlauf des 1. Dekanters aus dem Prozess ausgeschleust.

Die erfindungsgemäß hergestellte Kartoffelproteinfraktion vermeidet die oben beschriebenen Nachteile bekannter Kartoffelproteine (Farbe, Bitterkeit, Allergenität, Glycoalkaloide, antinutritiver Spaltprodukte, Eigengeschmack, Funktionslosigkeit) durch Auswaschen oder gemeinsame Abtrennung dieser Stoffe mit den Proteinen der ersten Stufe. Antinutritiv könnten einige Enzyme, hier sind wiederum herausragend die Proteaseinhibitoren, die bspw. in der Kartoffelknolle antibakterielle Funktionen haben, sein. Laut Literatur haben diese Enzyme ein geringes MG, so dass man als gesichert annehmen kann, dass diese nicht in der hier beschriebenen Fraktion enthalten sind. Darüber hinaus bewirkt die Wärmebehandlung eine Denaturierung und Inaktivierung, worauf auch die geringe Löslichkeit von 2 bis 5 % der Proteinfraktion hinweist.

Schadstoffe: Als Schadstoffe kommen bei Kartoffeln außer natürlich den "normalen" Umweltgiften wie Schwermetalle und Pestizide nur Glycoalkaloide (Solanin etc.) vor. Deren Abtrennung erfolgt in Stufe 1, wie oben beschrieben. Ein allergenes Potential derartiger Kartoffelproteine ist unbekannt und diese Kartoffelproteinfraktion eignet sich daher auch für die Produktion spezieller allergenfreier pflanzlicher Spezialnahrung und Kosmetika.

Gemäß SDS-PAGE betragen die Molekulargewichte der verschiedenen Proteine der erfindungsgemäßen Kartoffelproteinfraktion 14, 20, 22, 40, 97 kD. Die Fraktion besteht im wesentlichen aus Patatin (40 kD) und 20/22 kD, der Rest ist im Wesentlichen vernachlässigbar.

Die so hergestellte Kartoffelproteinfraktion wies folgende Eigenschaften auf:

max. 150 ppm Glycoalkaloide

max. 1% Stärke

max. 1 % Zucker

max. 1 % Rohfaser*

Isoelektrischer Punkt: etwa 5,4

pH-Wert von 4,0 bis 6,0,

max. 5% Asche:

Emulgierkapazität von 1:4:4 bis 1:4:6

Löslichkeit - 2% bis 5% (in Wasser bei Raumtemperatur und auch in heißem Wasser)

Wasserbindekapazität: 1:4 bis 1:5

Messung der Emulgierkapazität:

25 g Protein werden in 100 g Wasser mit einem Ultraturrax suspendiert. Danach gibt man unter weiterem Dispergieren langsam und portionsweise Öl dazu (z.B. Sonnenblumenkernöl, Rapsöl, Olivenöl usw.) bis die Emulsion bricht. Man gibt die Ölbindungs- oder Emulsionskapazität im Verhältnis der 3 Stoffe an mit der maximalen Ölbindung. 1: 4: 6 bedeutet, dass eine Mischung aus 1 Teil Protein und 4 Teilen Wasser 6 Teile Öl binden können, d.h. nach Zugabe von mehr als 150 g Öl in o.g. Suspension bricht die Testemulsion.

Wasserbindekapazitätsbestimmung:

5 g Protein werden in 95 g Wasser eingewogen und die Suspension 1 h gerührt. Danach wird zentrifugiert ( 20 min., 3.500 g), der Überstand vorsichtig abgegossen (u.U. den letzten Rest abpipettieren) und das nasse Protein ausgewogen.

$$(\text{Nassgewicht} - \text{Trockengewicht})/\text{Trockengewicht} = \text{Wasserbindekapazität}$$

Die Aminosäurenzusammensetzung der so erhaltenen Kartoffeleiweißfraktion mit einem Molekulargewicht zwischen 14 und 97 kD war (Schwankungen typisch für Naturprodukte):

| | |
|---|---|
| Ala | 3,7 - 3,9 |
| Arg | 3,9 bis 4,4 |
| Asp | 9,8 bis 12,6 |
| Cys | 1,4 bis 1,7 |
| Glu | 8,9 bis 9,5 |
| Gly | 4,7 bis 5,0 |
| His | 1,9 bis 2,3 |
| Ile | 4,9 bis 5,4 |
| Leu | 8,6 bis 9,6 |
| Met | 1,7 bis 1,9 |
| Phe | 5,8 bis 6,0 |
| Pro | 4,1 bis 4,5 |
| Ser | 4,3 bis 4,8 |
| Thr | 4,6 bis 5,2 |
| Try | 1,0 bis 1,2 |
| Tyr | 4,6 bis 5,0 |
| Val | 5,9 bis 6,9 |
| Lys | 7,3 bis 7,4 |

Essentielle Aminosäuren sind unterstrichen. Der Gesamtgehalt essentieller Aminosäuren ist 40,8 % bis 43,1 %. Die Summe der Aminosäuren in Trockensubstanz ist 95,6 %, in OS 91,5 %, Rohprotein (N*6,25) 85,4 % in der Trockensubstanz.

In dieser Kartoffeleiweißfraktion wird der hohe Nährwert des Kartoffelproteins den Menschen zugänglich gemacht und gleichzeitig Verarbeitern, die konfektionierte Lebensmittel herstellen, ein Protein zur Verfügung gestellt, das die erwähnten technologischen Vorteile bietet.

Bei höherer Temperatur wird das Protein übermäßig geschädigt und seine wertvollen Eigenschaften, die es für die Lebensmittelanwendung so nützlich machen werden zunehmend zerstört: neutraler Geschmack, helle Farbe, Löslichkeit, alle anderen Funktionalitäten ebenfalls, die Struktur wird verhornt und die Verdaulichkeit nimmt ab. Übrig bleibt im Kartoffelfruchtwasser die Proteinfraktion, die auch unter diesen Bedingungen löslich bleibt.

Herstellung von Kartoffeleiweiß

50 kg Kartoffeln der Sorte Saturna wurden gewaschen, in einer Mühle zerkleinert und das Kartoffelfruchtwasser durch Pressen der Reibsel in einer Zentrifuge unter Zusatz von Natriumbisulfit gewonnen. 25 I dieses Kartoffelfruchtwassers wurden mit Salzsäure auf einen pH-Wert von 4,6 gebracht und der ausfallende Niederschlag mit Proteinschlamm, Polyphenolen und Glycoalkaloiden wiederum abzentrifugiert. Der Überstand wurde 30 min auf 75°C erhitzt. Es entstand ein weißer Niederschlag, der in einer Zentrifuge abgetrennt wurde. Der Niederschlag wurde in zwei weiteren Waschschritten bei einer Temperatur von 70 °C mit Wasser gewaschen und 0,25 kg Trockensubstanz in Form eines hellen Pulvers erhalten.

Verwendung der Kartoffeleiweißfraktion als Emulgator in einer hypoallergenen Salatcreme

43,2 % Rüböl wurden mit 10 % Salzeigelb, 34,08% Wasser, 6,00 % Kartoffelprotein, 1,15% NaCl, 7,2% Zucker, 0,5% Kartoffelfaser, 0,03% Paprika, 0,01 % Carotin, 0,05% weißem Pfeffer, 7,14% 10%igen Branntweinessigs und 0,64 % scharfem Senf verrührt. Es entstand eine hypoallergene Salatcreme, in der Stärke und Eiweiß, die üblicherweise als Dickungsmittel und Emulgatoren verwendet werden, vermieden werden konnten, die eine gute Emulsionsstabilität sowie Haltbarkeit aufwies und geschmacklich nicht zu beanstanden war.

Verwendung der Kartoffeleiweißfraktion als Emulgator in hypoallergenem Tomatenketchup

30% 2-fach konzentriertes Tomatenmar,k, 35,4% Wasser, 9,5% 10%igen Branntweinessig, 19,00 % Zucker, 2,3% Salz, 2,5% Kartoffelprotein, 0,5% Kartoffelfaser und 0,8% Zitronensäure wurden verrührt. Hier wurde eine konservierungsmittelfreier Ketchup unter Austausch der sonst verwendeten Weizenstärke für Glutenallergiker erhalten.

Kohlenhydratreduzierter Nudelteig

150g Vollei, 400g Kartoffelprotein, 5 g Guarkernmehl, 100 g Wasser, 60g Kartoffelfasern und 6 g Salz wurden zu einem Nudelteig verknetet und in Nudeln umgeformt sowie getrocknet. Es wurde ein Produkt mit niedrigem Kohlenhydratgehalt, insbesondere mit wenig schnell absorbierbaren Kohlenhydraten erhalten, das sich für die Gewichtsreduktion sowie Diabetiker eignet.

Proteinangereicherte Karottencremesuppe

300g Karotten, 200g Kartoffeln, 40g Lauchzwiebeln, 15g Petersilie, 500g Wasser, 100g Kartoffelprotein, 10g Limonensaft, 250g Milch, 100 g saure Sahne, 2g schwarzer Pfeffer und 17 g Salz wurden zu einer Suppe von 1534 g verarbeitet. Diese Suppe eignet sich insbesondere als proteinangereicherte Aufbaukost.
Obwohl die Erfindung anhand bevorzugter Ausführungsbeispiele erläutert wurde, ist dem Fachmann ersichtlich, dass im Rahmen der Ansprüche mannigfaltige Abwandlungen derselben möglich sind, die ebenfalls unter den Schutzumfang fallen.

**Patentansprüche**

1. Verfahren zur Herstellung einer koagulierten Knollenfruchtproteinfraktion mit einem mittleren Molekulargewicht zwischen 14 kD bis 97 kD, **gekennzeichnet durch**:

   Vorlegen von Knollenfruchtsaft in wässriger Lösung;
   Fällen einer hochmolekulargewichtigen Knollenfruchtproteinfraktion, deren Hauptmenge ein Molekulargewicht von über 100 kD bis 600 kD besitzt, **durch** Einstellen eines sauren pH-Wertes von 2 - 7 und/oder einer Temperatur von 25 - 50°C und mechanisches Abtrennen der so ausgefällten Fraktion;
   Fällen einer mittelmolekularen koagulierten Knollenfruchtproteinfraktion in der Wärme **durch** Behandeln der nach der Abtrennung der hochmolekulargewichtigen Knollenfruchtproteinfraktion gewonnenen Lösung bei pH 2 bis 7, bevorzugt pH 3 bis 6, besonders bevorzugt pH 4 bis 5, und zwischen 60 bis 90°C, bevorzugt bei 80 °C und Mechanische Abtrennung der mittelmolekulargewichtigen koagulierten Knollenfruchtproteinfraktion mit einem Molekulargewicht zwischen etwa 14 kD bis 97 kD, wobei der Schwerpunkt der Molekulargewichtsverteilung zwischen 20 kD bis 60 kD liegt.

2. Verfahren nach Anspruch 1, ferner **gekennzeichnet durch** Waschen der so erhaltenen mittelmolekularen koagulierten Knollenfruchtproteinfraktion **durch** mindestens einmaliges Aufschlämmen mit Wasser bei neutralem oder bei einem sauren pH - Wert oberhalb des Fällungs-pH-Wertes, bei Raumtemperatur oder einer Temperatur unterhalb der Fällungstemperatur und mechanische Abtrennung der mittelmolekularen koagulierten Knollenfruchtproteinfraktion.

**3.** Verfahren nach Anspruch 2, **gekennzeichnet durch** Trocknen der gewaschenen Knollenfruchtproteinfraktion.

**4.** Verfahren nach einem der vorangehenden Ansprüche, **dadurch gekennzeichnet, dass** die mechanische Abtrennung mit einem Dekanter erfolgt.

**5.** Verfahren nach einem der vorangehenden Ansprüche , **dadurch gekennzeichnet, dass** die Trennschritte unter oxidationswidrigen Bedingungen durchgeführt werden, wie durch Zugabe eines Reduktionsmittels, wie Ascorbinsäure, Natriumbisulfit; Arbeiten unter Schutzgas und das Arbeiten in gasdichten Anlagen.

**6.** Knollenfruchtproteinfraktion mittleren Molekulargewichts, herstellbar nach einem Verfahren nach einem der vorangehenden Ansprüche, **gekennzeichnet durch** ein Molekulargewicht zwischen etwa 14 kD bis 97 kD, wobei der Schwerpunkt der Molekulargewichtsverteilung zwischen 20 kD bis 60 kD liegt

**7.** Knollenfruchtproteinfraktion nach Anspruch 5, **dadurch gekennzeichnet, dass** sie eine Kartoffeltproteinfraktion mit den nachfolgenden Parametern ist:

> pH: 4,0 bis 6,0
> Löslichkeit: 2 bis 8 % in Wasser bei Raumtemperatur

**8.** Verwendung der Knollenfruchtproteinfraktion nach einem der vorangehenden Ansprüche als Lebensmittel, Nahrungsmittelzusatz, Arzneimittelzusatz, Tierfutter, in der Kosmetik, als technisches Eiweiß, als Kleber.

**Claims**

**1.** Method for the production of a coagulated tuber protein fraction having a molecular weight between 14 kD and 97 kD, **characterized by**:

> providing tuber juice in aqueous solution;
> precipitating a tuber protein fraction **characterized by** a high molecular weight whose bulk has a molecular weight of more than 100 kD to 600 kD, by adjusting an acidic pH value of 2-7 and / or a temperature between 25 and 50 ° C and by a mechanical separation of the so precipitated fraction;
> precipitating a coagulated tuber protein fraction having a medium molecular weight in a warm environment by treating the solution obtained after the separation of the tuber protein fraction having a high molecular weight between pH 2 and 7, preferably between pH 3 and 6, most preferably between pH 4 and 5, and between 60 and 90 ° C, preferably at 80 ° C and
> mechanical separation of the coagulated tuber protein fraction having a medium molecular weight with a molecular weight between approximately 14 kD and 97 kD, wherein the center of gravity of the distribution of the molecular weight is between 20 kD and 60 kD.

**2.** Method according to claim 1, further **characterized by** the washing of the so obtained medium molecular weighed coagulated tuber protein fraction by at least one dispersion in water with a neutral or acidic pH value higher than the precipitation pH value, at room temperature or at a temperature lower than the precipitation temperature and mechanical separation of the coagulated tuber protein fraction having a medium molecular weight.

**3.** Method according to claim 2, **characterized by** the drying the washed tuber protein fraction.

**4.** Method according to one of the previous claims, **characterized in that** the mechanical separation is carried out by a decanter.

**5.** Method according to one of the previous claims, **characterized in that** the separation steps are carried out under non-oxidating conditions as by the addition of a reduction means like ascorbic acid, sodium bisulphite, working under protection gas and working in gas-proof installations.

**6.** Tuber protein fraction with medium molecular weight, to be produced by a method according to one of the previous claims, **characterized by** a molecular weight between approximately 14 kD and 97 kD, wherein the center of gravity of the distribution of the molecular weight is between 20 kD and 60 kD.

7. Tuber protein fraction according to claim 5, **characterized in that** it is a tuber protein fraction with one of the following parameters:

   pH: between 4,0 and 6,0
   Solubility: between 2 and 8 % in water at room temperature

8. Use of the tuber protein fraction according to one of the previous claims, as food, food additive, drug additive, animal food, in cosmetics, as technical protein, as adhesive.

**Revendications**

1. Méthode pour la production d'une fraction de protéine d'un tubercule coagulée ayant un poids moléculaire moyen entre 14 kD et 97 kD, **caractérisée par** :

   Soumission du jus du tubercule dans une solution aqueuse :

   Précipitation d'une fraction de protéine d'un tubercule ayant un poids moléculaire lourd de plus de 100 kD jusqu'à 600 kD, par le réglage d'une valeur pH acide de 2 - 7 et/ou d'une température entre 25 et 50 °C et une séparation mécanique de la fraction précipitée ;
   Précipitation d'une fraction de protéine d'un tubercule coagulée ayant un poids moléculaire moyen dans la chaleur en traitant la solution produite après la séparation de la fraction de protéine d'un tubercule ayant un poids moléculaire élevé avec une valeur pH entre 2 et 7, préférablement entre 3 et 6, très préférablement entre 4 et 5 et avec une température entre 60 et 90 ° C, préférablement de 80 °C et
   Séparation mécanique de la fraction de protéine d'un tubercule coagulée ayant un poids moléculaire moyen entre 14 kD et 97 kD, en étant le point de gravitation de la distribution du poids moléculaire entre 20 kD et 60 kD.

2. Méthode selon revendication 1, **caractérisée en outre par** le lavage de la fraction de protéine d'un tubercule coagulée obtenue et ayant un poids moléculaire moyen par un seul égorgement avec de l'eau avec une valeur pH neutre ou acide au-dessus de la valeur pH de précipitation, avec une température ambiante ou une température au-dessous de la température de précipitation ou de la séparation mécanique de la fraction de protéine d'un tubercule coagulée ayant un poids moléculaire moyen.

3. Méthode selon revendication 2, **caractérisée par** le séchage de la fraction de protéine d'un tubercule lavée.

4. Méthode selon une des revendications précédentes, **caractérisée en ce que** la séparation mécanique est exécutée par un décanteur.

5. Méthode selon une des revendications précédentes, **caractérisée en ce que** les passages de séparation sont exécutés dans des conditions contraires à l'oxydation, comme l'ajout d'un moyen de réduction, comme l'acide ascorbique, le bisulfite sodique, les travaux avec gaz de protection et le travail dans des installations étanches aux gaz.

6. Fraction de protéine d'un tubercule ayant un poids moléculaire moyen, qui peut être produite selon la procédure d'une des revendications précédentes, **caractérisée par** un poids moléculaire entre environ 14 kD et 97 kD, en étant le centre de gravitation de la distribution du poids moléculaire entre 20 kD et 60 kD.

7. Fraction de protéine d'un fruit bulbe selon revendication 5, **caractérisée en ce qu'**il s'agit d'une fraction de protéine d'une pomme de terre ayant les paramètres suivants :

   pH : 4,0 - 6,0
   Solubilité : 2 - 8 % dans l'eau avec la température ambiante

8. Utilisation d'une fraction de protéine d'un tubercule selon une des revendications précédentes, comme produits agro-alimentaires, additives agro-alimentaires, additives pour médicaments, alimentation animale, dans le secteur cosmétique, comme protéine technique et comme substance adhésive.